# EUROPEAN PATENT APPLICATION

(11) **EP 3 187 591 A1**
(43) Date of publication of application: **05.07.2017**
(21) Application number: 15828260.8
(22) Date of filing: 31.07.2015
(51) Int. Cl.: C12P 7/64, C12N 1/16, C12N 1/19, C12N 15/09, C12R 1/645

(54) **OIL/FAT-PRODUCING YEAST AND OIL/FAT PRODUCTION METHOD**

(30) Priority: 01.08.2014 JP 2014157746
(71) Applicant: Japan Science and Technology Agency, Kawaguchi-shi Saitama 332-0012 (JP)
(72) Inventor: SHIMA, Jun, Kyoto-shi Kyoto 612-8577 (JP); TANIMURA, Ayumi, Kyoto-shi Kyoto 606-8501 (JP); TAKASHIMA, Masako, Wako-shi Saitama 351-0198 (JP); ENDO, Rikiya, Wako-shi Saitama 351-0198 (JP); OHOKUMA, Moriya, Wako-shi Saitama 351-0198 (JP); SUGITA, Takashi, Kiyose-shi Tokyo 204-8588 (JP)
(74) Representative: Bachinger-Fuchs, Eva-Maria
(86) International application number: PCT/JP2015/003862
(87) International publication number: WO 2016/017183

(57) **Abstract**

The Rhodosporidium toruloides IPM33-18 strain (NITE BP-01900) or a genetically modified variant thereof is provided as a novel yeast that is useful for producing oils from biomass. Further, a method for producing oils, comprising the steps of culturing the IPM33-18 strain or the like in a medium containing a carbon source to allow oils to accumulate in a culture, and collecting the oils from the culture is provided. According to this production method, the oils can be collected from a liquid fraction of the culture.

## Description

### Technical Field

The present invention relates to an oil-producing yeast and a method for producing oils. In more detail, the present invention relates to a yeast strain that accumulates oils outside the yeast cells and a method for producing oils using the same.

### Background Art

There is a demand for the utilization of biomass, which is a carbon-neutral resource, in an attempt to break dependence on fossil fuel and to suppress greenhouse gas emission. In regard to the utilization of biomass, Patent Literature 1 discloses a technology for producing oils using algae such as Chlorella. The technology disclosed in this literature involves blasting a mixture containing algae and water in a pressure vessel, whereby oils are collected from algae without using an organic solvent.

In connection with the present invention, Patent Literature 2 discloses yeasts belonging to the genus Trichosporon that produce oils from carbohydrates.

### Citation List

### Patent Literature

[Patent Literature 1] Japanese Patent Laid-Open No. 2012-5398
[Patent Literature 2] Japanese Patent Laid-Open No. 7-236492

### Summary of Invention

### Technical Problem

The yeasts belonging to the genus Trichosporon disclosed in Patent Literature 2 can produce oils extracellularly, allowing oils to be collected without requiring operations to extract oils from within the yeast cells. Thus, the above yeasts may be utilized for the production of oils from biomass. However, the sugar assimilability of the yeasts belonging to the genus Trichosporon have not yet been studied in detail. Further, because the yeasts belonging to the genus Trichosporon have a reproduction process similar to that of filamentous fungi, many of them exhibit slow growth. Moreover, some species are pathogenic.

A main object of the present invention is to provide a novel yeast that is useful for producing oils from biomass.

### Solution to Problem

In order to solve the aforementioned problem, the present invention provides the following items [1] to [4].
[1] A method for producing oils, comprising the steps of:
   culturing a Rhodosporidium toruloides IPM33-18 strain (NITE BP-01900) or a genetically modified variant thereof in a medium containing a carbon source to allow oils to accumulate in a culture, and
   collecting the oils from the culture.
[2] The production method according to [1], wherein the method comprises the step of collecting oils from a liquid fraction of the culture.
[3] The production method according to [1] or [2], wherein the method does not comprise the step of extracting oils from a yeast cell.
[4] The production method according to [1] or [2], wherein the method comprises the step of collecting oils from a yeast cell.
[5] The production method according to any of [1] to [4], wherein the genetic modification is an introduction of a 2-deoxyglucose resistance mutation.
[6] A Rhodosporidium toruloides IPM33-18 strain (NITE BP-01900) or a genetically modified variant thereof.

In the present invention, the terms "medium", "culture", and "liquid fraction of a culture" are used in the following senses.

A "medium" refers to a liquid containing nutrient components necessary for the growth of yeasts. A "medium" does not contain either yeast cells or oils produced by the yeast cells.

A "culture" contains a medium and yeast cells grown in the medium. Further, a "culture" contains oils produced by yeast cells grown in a medium.

A "liquid fraction of a culture" refers to a yeast cell-free liquid phase of a culture. A "liquid fraction of a culture" contains oils produced by yeast cells that have accumulated in the liquid fraction. That is, oils produced by yeast cells that have accumulated within the yeast cells are not contained in a "liquid fraction of a culture." Note that a "liquid fraction of a culture" may be composed of an aqueous layer containing a medium and an oil layer containing oils produced in a liquid fraction by yeast cells.

Further, in the present invention, the phrase that "oils accumulate in a culture" means that oils produced by yeast cells accumulate within the "yeast cells" as well as in a "liquid fraction of a culture." Of these, the phrase "accumulate in a liquid fraction of a culture" encompasses the release of oils produced by yeasts into the liquid fraction, which encompasses the case in which yeasts release the oils they produced to the outside of the yeast cells through secretion and the case in which yeasts that have produced oils within the yeast cells undergo cell death, followed by disruption, whereby the oils are released to the outside of the yeast cells. Note that the phases "accumulate outside the yeast cells" and "accumulate in a liquid fraction of a culture" are used synonymously.

### Advantageous Effects of Invention

The present invention provides a new yeast that is useful for producing oils from biomass.

### Brief Description of Drawings

[Figure 1] Figure 1 is a graph illustrating the results of the measurement of the amount of oils accumulated in and out of the yeast cells of the IPM33-18 strain (Example 1).
[Figure 2] Figure 2 is a graph illustrating the results of the measurement of the concentration of glucose in the culture supernatants of the IPM33-18 strain (Example 1).
[Figure 3] Figure 3 is a graph illustrating the results of the measurement of the concentration of nitrogen in the culture supernatants of the IPM33-18 strain (Example 1).
[Figure 4] Figure 4 is a graph illustrating the results of the measurement of the weight of the yeast cells (dry weight) of the IPM33-18 strain (Example 1).
[Figure 5] Figure 5 is a graph illustrating the composition of fatty acid in oils accumulated outside the yeast cells of the IPM33-18 strain on Day 10 of culture (Example 1).
[Figure 6] Figure 6 is a graph illustrating the results of the measurement of the amount of oils accumulated outside the yeast cells of a genetically modified variant of the IPM33-18 strain (Example 3).

### Description of Embodiments

Hereinbelow, preferred embodiments of the present invention will be described. Note that the embodiments described below are intended to show an example of representative embodiments of the present invention, and they should not be used to interpret the scope of the present invention narrowly.

The present inventors isolated yeast strains from soils and plants in the subtropical region (Iriomote Island) and the cold region (Rishiri Island) of Japan to construct a library consisting of 1000 or more strains of yeast (PLOS ONE, 2012, Vol. 7, No. 11, e50784). As a result of screening the above yeast library for a strain capable of accumulating oils outside the yeast cells, they newly found that the Rhodosporidium toruloides IPM33-18 strain had such an ability. Based on this finding, the present invention provides a method for producing oils using the above strain.

The IPM33-18 strain has been internationally deposited at National Institute of Technology and Evaluation, Patent Microorganisms Depositary (Room No. 122, 2-5-8 Kazusakamatari, Kisarazu-shi, Chiba) under the deposition No. NITE BP-01900.

The IPM33-18 strain grows at a temperature of 10 to 37°C and assimilates various types of sugar. Therefore, the above strain can be utilized to produce oils from a wide variety of biomasses under normal environments. Further, unlike common oleaginous yeasts, which accumulate oils they produced within the yeast cells (oil-accumulating yeasts), the IPM33-18 strain accumulates oils they produced outside the yeast cells. Therefore, by using the above strain, oils can be produced without performing operations to extract oils from within the yeast cells.

The mycological properties of the IPM33-18 strain are as follows.

### [Taxonomic position]

Rhodosporidium toruloides

### [Scientific properties]

The base sequences of the D1/D2 regions of genomic DNA are consistent with those of the standard strain of the above species. With regard to properties such as the morphology in media, the IPM33-18 strain shows similar properties to Rhodosporidium toruloides.

### [Culture condition]

Aerobic, static culture

**Medium composition**

| | |
|---|---|
| Glucose | 10.0 g |
| Peptone | 5.0 g |
| Yeast extract | 3.0 g |
| Malt extract | 3.0 g |
| Agar | 20.0 g |
| Distilled water | 1.0 L |

Also, in the present invention, the IPM33-18 strain can be replaced by or used in combination with a genetically modified variant thereof. For gene modification, techniques that have been conventionally known to those skilled in the art such as culturing the IPM33-18 strain with the use of a mutagenic substance and a gene transfer method using a vector can be selected as appropriate.

As for gene modification, such a modification that improves sugar assimilability or increases the amount of oils accumulated in a culture is preferable. Examples of the modification that improves sugar assimilability include the introduction of a 2-deoxyglucose resistance mutation.

Alternatively, in the present invention, an extract of the IPM33-18 strain can be used in place of the IPM33-18 strain. As for the extract, it is also possible to purify the desired enzyme and use a portion or all of the resulting product. For example, when the use of lipase derived from the IPM33-18 strain is desired, it can be purified from an ammonium sulfate fraction in accordance with the method described in the literature of lukaszewicz et al. (1st Annual International Interdisciplinary Conference, AIIC 2013, 24 to 26 April, Azores, Portugal, pages 441 to 449).

The method for producing oils of the present invention includes the steps of culturing the Rhodosporidium toruloides IPM33-18 strain or a genetically modified variant thereof in a medium containing a carbon source to allow oils to accumulate in a culture and collecting the oils from the culture. As will be described later, the step of collecting oils from a culture may be either the step of collecting oils from a liquid fraction of a culture or the step of collecting oils from a liquid fraction as well as from the yeast cells.

Culture of the IPM33-18 strain can be carried out by a conventionally known technique using a culture medium containing a carbon source. Because the IPM33-18 strain can assimilate various types of sugar, as the carbon source, sugars, sugar alcohol, and acidic sugar as well as biomasses containing these substances can be used without particular limitation. At this point, in the present invention, a "biomass" refers to a renewable material containing the aforementioned carbon sources.

Examples of the sugars include monosaccharides, oligosaccharides, and polysaccharides. Oligosaccharides refer to di- to decasaccharides, which may be homooligosaccharides or heterooligosaccharides. Also, polysaccharides refer to sugars having a greater number of monosaccharide unit than oligosaccharides, which may be homopolysaccharides or heteropolysaccharides. Specific examples of the monosaccharides include a pentose such as L-arabinose, D-xylose, and D-ribose, a hexose such as D-glucose, D-galactose, D-fructose, and D-mannose, and a 6-deoxyhexose such as L-rhamnose. Examples of the oligosaccharides include disaccharides such as sucrose, maltose, lactose, cellobiose, trehalose, and melibiose and trisaccharides such as raffinose. Examples of the polysaccharides include starch, cellulose, glycogen, dextran, mannan, and xylan. The above sugars can be used alone or in an appropriate combination. The aforementioned combination also includes starch hydrolysate and the like. Also, as for sugars, a raw material containing sugars as the main component such as molasses and soybean curd residue can also be used.

Examples of the sugar alcohol include D-sorbitol, D-mannitol, galactitol, and maltitol. Examples of the acidic sugar include glucuronic acid and galacturonic acid.

Although the amount of a carbon source in a medium is not limited, normally, it is about 3 to 15 %(w/w).

A medium can contain a nitrogen source, an inorganic substance, and other nutrients in addition to a carbon source. As for the nitrogen source, an inorganic or organic nitrogen compound such as ammonia, ammonium chloride, ammonium sulfate, ammonium carbonate, ammonium acetate, sodium nitrate, and urea can be used. Further, as for the nitrogen source, a nitrogen-containing naturally occurring substance such as peptone, a meat extract, a yeast extract, corn steep liquor, casein hydrolysate, fishmeal or its digested product, and defatted soybean cake or its digested product can also be used. As for the inorganic substance, monopotassium phosphate, dipotassium phosphate, magnesium sulfate, sodium chloride, ferrous sulfate, ferrous chloride, manganese sulfate, calcium chloride, calcium carbonate, zinc sulfate, copper sulfate, boric acid/ammonium molybdate, and potassium iodide can be used.

Culture is carried out under aerobic conditions such as shaking culture or submerged stirring culture. The culture temperature is normally preferably 20 to 35°C; however, other temperature conditions can also be used as long as yeasts can grow under those temperature conditions. The pH of a medium during culture is normally 4.0 to 7.2. Oils are produced and accumulated outside the yeast cells normally from two to four days after the initiation of culture.

Oils can be collected from a culture by a conventionally known technique using a lipophilic solvent. Specifically, a solvent is added to a culture so that oils contained in a liquid fraction of the culture are collected in the solvent. Collection of oils can also be performed by separating yeast cells from a culture to obtain a liquid fraction, and then adding a solvent to the liquid fraction. A liquid fraction of a culture can be obtained by separating yeast cells from a culture by operations such as centrifugation and sedimentation or devices such as a separator, a decanter, and a filtration device. Also, oils accumulated within the yeast cells can also be extracted in accordance with a routine method. As for the solvent, an organic solvent that stays liquid at normal temperature, which can dissolve oils but is not or poorly miscible with water, for example, a halogenated lower alkane (chloroform, methylene chloride, carbon tetrachloride, and 1,2-dichloroethane), n-hexane, ethyl ether, ethyl acetate, and an aromatic hydrocarbon (benzene, toluene, and xylene) are preferably used. The amount of an extraction solvent added is not particularly limited as long as it is added in such an amount that can fully collect oils produced and accumulated in a culture or a liquid fraction thereof.

Examples of the oils obtained by the production method of the present invention include an aliphatic ester compound composed of aliphatic carboxylic acid and aliphatic alcohol. Aliphatic carboxylic acid is not particularly limited as long as it is produced by yeasts, and examples thereof include aliphatic carboxylic acid having 8 to 24, preferably 12 to 24 carbon atoms, and specific examples thereof include myristic acid, palmitic acid, palmitoleic acid, stearic acid, oleic acid, linoleic acid, linolenic acid, behenic acid, and lignoceric acid. Also, the oils may contain a phospholipid, a free fatty acid, a glycolipid, a steroid compound, and a photosynthetic pigment such as carotenoid.

According to the production method of the present invention, oils can be collected from a culture or a liquid fraction of the culture without performing operations to extract oils from within the yeast cells. Conventionally, operations to disrupt rigid cell walls have been required to extract oils within the yeast cells. In contrast, according to the production method of the present invention, oils can be collected merely by mixing a culture or a liquid fraction of a culture and a lipophilic solvent. Also, as described earlier, the IPM33-18 strain of the present invention can assimilate various types of sugar. Therefore, the present invention is useful for producing oils from biomass, and for example, the present invention can be applied to biodiesel production and biorefinery utilizing unused biomass.

### Examples

### <Example 1: The ability of the IPM33-18 strain to accumulate oils outside the yeast cells>

The IPM33-18 strain was cultured and oils were collected from a liquid fraction of the culture in accordance with the following procedure.

### [Culture]

Culture was conducted under the following conditions.

Colonies on an YM agar medium were scraped with a platinum loop and transplanted in a 3 ml YM medium, followed by preculture at 27°C and 150 rpm overnight. The absorbance at 600 nm was measured with a spectrophotometer to determine turbidity (OD₆₀₀). The preculture liquid thus obtained was seeded in a 25 ml SS2 medium (3% glucose, 0.5% ammonium sulfate, 0.05% magnesium sulfate, 0.01% sodium chloride, 0.01% calcium chloride, and 0.01% yeast extract) so as to achieve an OD₆₀₀ of 0.2, followed by main culture at 27°C and 150 rpm.

### [Sampling]

On Days 2, 4, 6, 8, and 10 of main culture, 3 ml of cultures were collected and subjected to centrifugation at 12,000 rpm for five minutes to collect yeast cells. Culture supernatants (liquid fractions of the cultures) were used to measure the concentrations of glucose and nitrogen. The yeast cells were washed once with distilled water and subjected to centrifugation once again, followed by freeze drying.

Also, 5 mL of hexane was added to the residual amount of culture supernatants, followed by stirring, and the resulting upper layer (hexane layer) fractions were collected. Hexane was removed by an evaporator to obtain red oily residues.

On Days 2, 4, 6, 8, and 10 of culture, measurements were taken with respect to the following items.

### [Glucose concentration]

The concentration of glucose in the culture supernatants was measured by a high-performance liquid chromatograph. High-performance liquid chromatographic analysis was conducted under the following conditions.
High-performance liquid chromatograph: Prominence, the product of Shimadzu Corporation
Column: Aminex Fermentation Monitoring Column, the product of Bio-Rad Laboratories, Inc.
Guard column: Micro-Guard Cation H Refill Cartridges, the product of Bio-Rad Laboratories, Inc.
Detector: RID 10A, the product of Shimadzu Corporation
Column oven: CTO 20A, the product of Shimadzu Corporation
Autosampler: SIL-20A, the product of Shimadzu Corporation
Mobile phase: 5 mM Aqueous solution of sulfuric acid, 0.6 mL/min
Column temperature: 60°C

Glucose was identified and its concentration was determined from the relative retention value and area of peak of a known concentration of D-glucose (Wako Pure Chemical Industries, Ltd.)

### [Nitrogen concentration]

The concentration of nitrogen in the culture supernatants was measured in accordance with the method described in a literature (Bioresour. Technol., 2012, Vol. 114, pp. 443 to 449). To 100 µl of supernatants, 500 µl of an alkaline hypochlorite solution, 500 µl of a phenol nitroprusside solution, and 3 ml of water were added. After leaving to stand at 25°C for one hour, the absorbance at 570 nm was measured. The concentration of nitrogen was determined based on a calibration curve created using 20, 40, and 80 mg/l aqueous solutions of ammonium sulfate.

### [Amount of oils]

The dried yeast cells and red oily residues obtained by sampling as described above were methylated in accordance with the method described in a literature (J. Lipid Res, 2010, Vol. 51, No. 3, pp. 635 to 640). After adding 300 µl of toluene and 1.5 ml of methanol to the dried yeast cells or red oily residues, only the dried yeast cells were subjected to 15 minutes of sonication. Subsequently, 300 µl of 85% methanol containing 8% hydrochloric acid was added, followed by stirring, and the samples were left to stand overnight at 45°C. Lastly, 1 mL of hexane and 1 mL of distilled water were added, followed by stirring, and the resulting upper layer (hexane layer) fractions were obtained and subjected to the following gas chromatographic analysis.

The composition of fatty acid methyl esters was analyzed by gas chromatographic analysis. The gas chromatographic analysis was conducted under the following conditions.
Gas chromatograph: GC-2010 Plus, the product of Shimadzu Corporation
Detector: FID
Autosampler: AOC20, the product of Shimadzu Corporation
Column: DB-23 Capillary column (30 m x 0.25 mm x 0.25 µm), the product of Agilent Technologies
Temperature program: Keep at 50°C for 2 minutes, heat to 180°C by 10°C per minute, keep there for 5 minutes, and heat to 240°C by 5°C per minute, and keep there for 3 minutes.
Carrier gas: Helium (1 mL per minute)
Make up gas: Nitrogen
Injector temperature: 250°C
Detector temperature: 300°C
Split ratio: 50 : 1

From the relative retention values of the standard substances (methyl arachidate, methyl behenate, methyl decanoate, methyl cis-13-docosenoate, methyl dodecanoate, methyl linoleate, methyl linolenate, methyl myristate, methyl octanoate, methyl oleate, methyl palmitate, methyl palmitoleate, methyl stearate, and methyl tetracosanoate, Wako Pure Chemical Industries, Ltd.), each fatty acid methyl ester was identified. From the area of peak of a fatty acid methyl ester mix (18918-1AMP, Supelco), the concentration of each fatty acid methyl ester was determined.

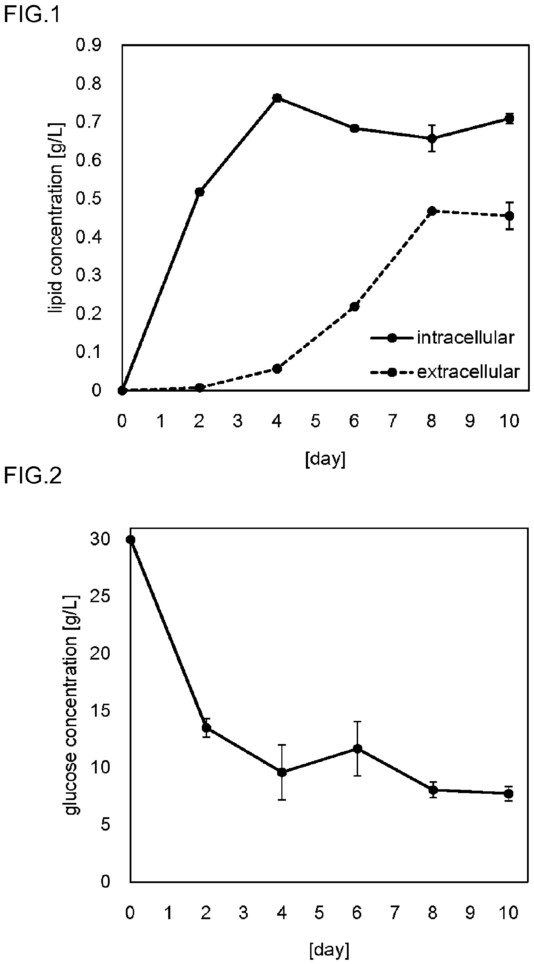

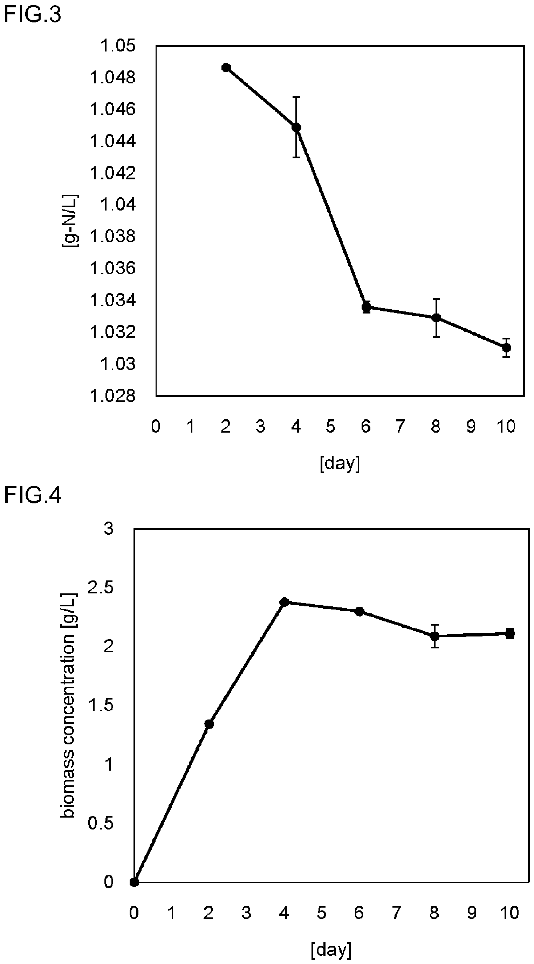

The changes in the amount of oils accumulated in and out of the yeast cells during the culture period are shown in Figure 1. On the vertical axis in Figure 1, the amount of oils indicates the amount of oils accumulated within the yeast cells contained in 1 L of a culture (intracellular) and the amount of oils accumulated in liquid fractions (extracellular). Also, the changes in the concentrations of glucose and nitrogen in culture supernatants and in the weight of dry yeast cells are shown in Figures 2 to 4.

It was revealed that while glucose and nitrogen in the medium were consumed, oils were produced, and a certain amount of oils thus produced accumulated outside the yeast cells as culture proceeded. Particularly after Day 4, on which the proliferation of yeast cells reached a plateau, the extracellular accumulation of oils increased, resulting in a marked increase in the ratio of the amount of extracellular accumulation to the amount of intracellular accumulation.

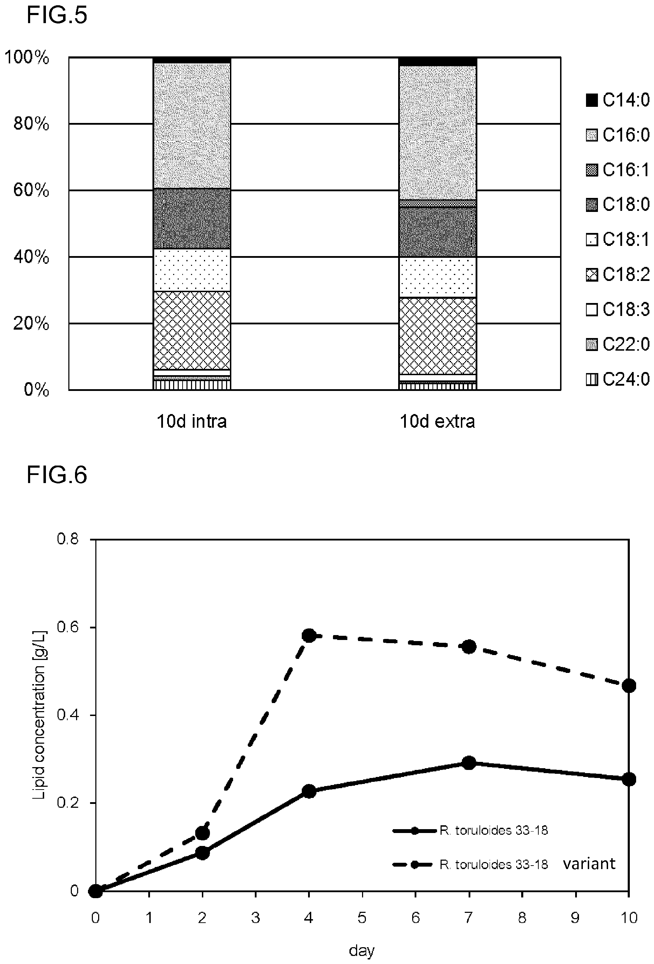

The fatty acid composition of oils accumulated outside the yeast cells (Day 10 of culture) is shown in Figure 5. There was no noticeable difference between the composition of oils accumulated outside (10d extra) and inside (10 intra) the yeast cells, and in both cases major fatty acids such as myristic acid (C14 : 0), palmitic acid (C16 : 0), palmitoleic acid (C16 : 1), stearic acid (C18 : 0), oleic acid (C18 : 1), linoleic acid (C18 : 2), linolenic acid (C18 : 3), behenic acid (C22 : 0), and lignoceric acid (C24 : 0) were contained.

### <Example 2: Sugar assimilability of the IPM33-18 strain>

The sugar assimilability of the IPM33-18 strain was analyzed by replacing glucose used as the carbon source in Example 1 by other sugars and detecting the extracellular accumulation of oils. As for the sugars, xylose, arabinose, glycerol, cellobiose, starch, and xylan were used. As a result, similarly to glucose, the extracellular accumulation of oils was confirmed when any of the above sugars was used as the carbon source, revealing that the IPM33-18 strain can assimilate various types of sugar.

### <Example 3: Genetic modification of the IPM33-18 strain>

A 2-deoxyglucose resistance mutation was introduced into the IPM33-18 strain and the changes in the amount of oils accumulated outside the yeast cells was analyzed.

In accordance with the method described in a literature (Appl. Microbiol. Biotechnol. 2011, Vol. 90, No. 4, pp. 1573 to 1586), a 2-deoxyglucose resistance mutation was introduced into the IPM33-18 strain to create a genetically modified variant. The resulting genetically modified variant was cultured in a synthetic xylose medium (0.17% YNB, 0.5% AS, and 6% Xylose). The culture conditions were the same as those used in Example 1, except the medium.

The changes in the amount of oils accumulated outside the yeast cells during the culture period are shown in Figure 6. The amount of oils produced was markedly increased by the introduction of a 2-deoxyglucose resistance mutation.

| | | |
|---|---|---|
| PCT | | |
| 0-1 | Form PCT/RO/134 | |
| | Indications relating to deposited microorganism or other biological material Rule created the (PCT 13bis) was by electronic filing software PCT-SAFE Version 3.51.064.240 MT/FOP 20141001/0.20.5.21. | |
| 0-1-1 | | PCT-SAFE |
| | | Version 3.51.064.240 MT/FOP 20141001/0.20.5.21 |
| 0-2 | International application No. | |
| 0-3 | Applicant's or agent's file reference | PJS-9021WO |
| | | |
| 1 | The indications made below relate to the microorganism or other biological material referred to in the Detailed Description of the Invention. | |
| 1-1 | Paragraph Number | 0014 |
| 1-3 | Indication of deposit | |
| 1-3-1 | Name of depositary institution | NPMD, National Institute of Technology and Evaluation, Patent Microorganisms Depositary (NPMD) |
| 1-3-2 | Address of depositary institution (including postal code and country) | Room No. 122, 2-5-8 Kazusakamatari, Kisarazu-shi, Chiba, 292-0818 Japan |
| 1-3-3 | Date of deposit | July 16, 2014 (16.07.2014) |
| 1-3-4 | Accession Number | NPMD NITE BP-01900 |
| 1-4 | Additional indications | None |
| 1-5 | Designated states for which indications are made | All designated States |
| 1-6 | Separate furnishing of indications | None |
| | The indications listed right will be submitted to the International Bureau later | |
| For receiving Office use only | | |
| 0-4 | This sheet was received with the international application | |
| | (Yes/No) | |
| 0-4-1 | Authorized officer | |
| For International Bureau use only | | |
| 0-5 | This sheet was received by the International Bureau on: | |
| 0-5-1 | Authorized officer | |

## Claims

1. A method for producing oils, comprising the steps of:
culturing a Rhodosporidium toruloides IPM33-18 strain (NITE BP-01900) or a genetically modified variant thereof in a medium containing a carbon source to allow oils to accumulate in a culture, and
collecting the oils from the culture.

2. The production method according to claim 1, wherein the method comprises the step of collecting oils from a liquid fraction of the culture.

3. The production method according to claim 1 or 2, wherein the method does not comprise the step of extracting oils from a yeast cell.

4. The production method according to claim 1 or 2, wherein the method comprises the step of collecting oils from a yeast cell.

5. The production method according to any one of claims 1 to 4, wherein the genetic modification is an introduction of a 2-deoxyglucose resistance mutation.

6. A Rhodosporidium toruloides IPM33-18 strain (NITE BP-01900) or a genetically modified variant thereof.
